# EUROPEAN PATENT SPECIFICATION

(11) **EP 3 901 610 B1**
(45) Date of publication and mention of the grant of the patent: **25.06.2025**
(21) Application number: 21168662.1
(22) Date of filing: 15.04.2021
(51) Int. Cl.: G01N 7/14, G01N 15/06, G01N 9/00, G01N 33/18

(54) **ASSAY ROBOT FOR MEASURING UNRESOLVED GASES IN A LIQUID**
TESTROBOTER ZUM MESSEN UNGELÖSTER GASE IN EINER FLÜSSIGKEIT
ROBOT POUR MESURER LES GAZ NON DISSOUS DANS UN LIQUIDE

(30) Priority: 24.04.2020 DK PA202000483
(43) Date of publication of application: 27.10.2021
(73) Proprietor: Zürcher, Jacob, 4700 Naestved (DK)
(72) Inventor: HANSEN, Niels Flemming Greve, 4400 Kalundborg (DK)
(74) Representative: Sun, Yiming

(56) References cited:
- EP-A2- 1 445 611
- WO-A1-2004/034035
- CN-B- 101 699 295
- US-B1- 6 847 898

## Description

### TECHNICAL FIELD OF THE INVENTION

The invention disclosed herein generally relates to a device for measuring unresolved gases in liquids. More particularly, the present invention relates to an assay robot for efficiently measuring the amount of residual unresolved gas relative to the liquid in real-time using a sensor and treating a suspension of particles/solid contents in the carrier liquid.

### BACKGROUND

Solid particles of small dimensions contained in liquids, especially when they float or are suspended therein, are generally surrounded in or encased by numerous minute air bubbles. The adhesion of the air bubbles to the particles makes it difficult to remove them and sometimes impossible by the ordinary methods. The smaller the bubbles, the greater the difficulty. This is particularly the case in handling washing waters from mines, white waters from paper mills and generally all effluents in which solids are contained or suspended in a finely divided state.

Numerous methods and pieces of equipment have been devised to separate liquids from each other and to separate any gas that may be entrapped in the liquids. Such apparatus and methods have wide application in many industries. In addition, the measurement of the concentrations of unresolved gases and volatile components in liquids is very helpful for process control and to indicate and prevent process instabilities in many chemical and biotechnological plants.

A prior art, JPS5388792 of NISHIHARA KANKIYOU EISEI KENKI*,* discloses an assay robot for measuring unresolved gases, wherein said measuring unresolved gases comprises condensing air bubbles in the liquid, and wherein said condensing air bubbles comprises providing an assay robot comprising valves securely disposed in the housing for distributing a sample liquid/product and water into a cylinder. Further, the assay robot comprises a pressure chamber, configured to increase the pressure of the liquid above the equilibrium level, thereby condensing air bubbles in the liquid. However, above-mentioned prior art fails to provide solution on providing a contained assay robot combining a measurement of unresolved gases relative to the liquid.

Another device for measuring unresolved gases in a liquid similar to the device mentioned above can be found in US6847898B1.

However, the prior devices and methods suffer from certain disadvantages such as being limited in measuring the amount of residual unresolved gas relative to the liquid in real-time and they cannot satisfactorily separate the liquid from the particles. Further, the development of a precise and efficient measuring system is very challenging because air bubbles are dynamic and unstable relative to time and space in a moving liquid, thus hindering an accurate validation of the measuring system. Further, the air bubbles may remain in the liquid, causing an inaccurate or variable reading.

In the light of above-mentioned problems, there is a need for an assay robot for measuring bubbling liquid with particles or high solid contents by comminuting and homogenizing the particles in the liquid to optimize the repeatability of the measurement result. Further, there is a need for an assay robot that prepares the fluid for online analysis, using a standard optical sensor, for example, a turbidity sensor.

### SUMMARY OF THE INVENTION

This summary is provided to introduce a selection of concepts in a simplified form that is further disclosed in the detailed description of the invention. This summary is not intended to identify key or essential inventive concepts of the claimed subject matter, nor is it intended for determining the scope of the claimed subject matter. The invention is defined by the appended claims.

The present invention discloses a device for measuring unresolved gases in liquids. More particularly, the present invention relates to an assay robot for efficiently measuring the amount of residual unresolved gas relative to the liquid in real-time using a sensor.

The robot is configured to measure the unresolved gases relative to the liquid. The robot comprises a housing for supporting components of the robot. In one embodiment, the housing is securely mounted on one or more supports from a ground level. In another embodiment, the housing is rotatably mounted on one or more supports from a ground level. The one or more supports are securely anchored to the ground using an anchor. In one embodiment, a pair of handles are securely affixed to both sides of the housing. In one embodiment, a screen is securely mounted on a front portion of the housing for displaying the measured value of the unresolved gases relative to the liquid.

The components of the robot are securely and operatively disposed within the housing. The components include, but not limited to, one or more valve, cylinders, a homogenizer, a holding cell, a pressure chamber, a pneumatic cylinder, and a sensor. The valves are securely disposed in the housing for distributing a sample liquid/product and water into the cylinders. The homogenizer is securely and fluidly connected to the cylinders and is configured to receive and homogenize the sample liquid and water and at the same time, the liquid is diluted in a mixing ratio determined by the stroke length of the cylinders.

The holding cell is securely connected to the homogenizer via a hose. The holding cell is configured to exhaust the liquid for removing large bubbles. In one embodiment, the holding cell comprises a spiral, which is configured to skim the liquid before running down to the sensor. In one embodiment, the holding cell further comprises a needle valve, which is configured to regulate holding time for exhausting the liquid. The pressure chamber is connected to the holding cell and is configured to increase the pressure of the liquid above the equilibrium level, thereby condensing air bubbles in the liquid. The pneumatic cylinder is operatively connected to the pressure chamber. The pneumatic cylinder further comprises a piston, which is configured to move to and fro within the pressure chamber, thereby measuring the unresolved gases relative to the liquid based on the volume displaced by the piston in the pressure chamber.

In one embodiment, the sensor is securely and fluidly connected to a bottom portion of the pressure chamber, configured to detect the volume displaced by the piston in the pressure chamber and log or record the unresolved gases relative to the liquid. In one embodiment, the logged unresolved gases relative to the liquid could be displayed on the screen, which is mounted on the housing. In one embodiment, the sensor could be, but not limited to, an optical sensor and a magnetic inductive sensor.

In one embodiment, the surface tension of the liquid squeezes the air bubbles with a radius of a few hundred nanometers so hard that the pressure in the air bubble could withstand a pressure difference of about 15 bar. Therefore, the robot raises the pressure to a difference of more than 35 bar for 10 seconds before logging the measurement value. The robot comminutes and homogenizes the particles in the liquid to optimize the repeatability of the measurement result. Liquid with uniform particles gives the highest data quality.

Other objects, features and advantages of the present invention will become apparent from the following detailed description.

### BRIEF DESCRIPTION OF THE DRAWINGS

The foregoing summary, as well as the following detailed description of the invention, is better understood when read in conjunction with the appended drawings. For illustrating the invention, exemplary constructions of the invention are shown in the drawings. However, the invention is not limited to the specific methods and structures disclosed herein. The description of a method step or a structure referenced by a numeral in a drawing is applicable to the description of that method step or structure shown by that same numeral in any subsequent drawing herein.
FIG. **1** exemplarily illustrates a front view of an assay robot securely and rotatably mounted on one or more supports in an embodiment of the present invention.
FIG. **2** exemplarily illustrates a side view of the assay robot securely and rotatably mounted on one or more supports in one embodiment of the present invention.
FIG. **3** exemplarily illustrates a rear perspective view of components of the assay robot in one embodiment of the present invention.
FIG. **4** exemplarily illustrates a front perspective view of the components of the assay robot in one embodiment of the present invention.
FIG. **5** exemplarily illustrates a rear perspective view a water heater securely disposed in the housing in one embodiment of the present invention.
FIG. **6** exemplarily illustrates a front perspective view the water heater securely disposed in the housing in one embodiment of the present invention.
FIG. **7** exemplarily illustrates a top perspective view of valves and hose connections in one embodiment of the present invention.
FIG. **8** exemplarily illustrates a side perspective view of the valves and hose connections in one embodiment of the present invention.
FIG. **9** exemplarily illustrates an exploded view of a rotor rotatably securing to a shaft of a motor in one embodiment of the present invention.

### DETAILED DESCRIPTION OF THE INVENTION

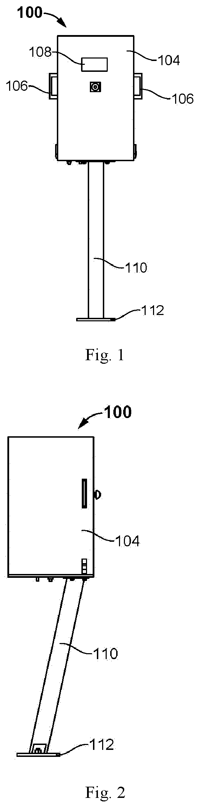

Referring to FIGs. **1-2****,** an assay robot **100** for measuring unresolved gases in a liquid or high particle aqueous solution is disclosed. The robot **100** is configured to measure the amount of unresolved gases relative to the liquid. The robot **100** comprises a housing **104** for supporting multiple components of the robot **100.** In one embodiment, the housing **104** is securely mounted on one or more supports **110** from a ground level. In another embodiment, the housing **104** is rotatably mounted on one or more supports **110** from a ground level as shown in FIG. **2****.** The one or more supports **110** are securely anchored to the ground using an anchor **112.** In one embodiment, a pair of handles **106** are securely affixed to both sides of the housing **104.** In one embodiment, a screen **108** is securely mounted on a front portion of the housing **104** for displaying the measured value of the unresolved gases relative to the liquid. In one example, the robot **100** is configured to measure the turbidity value i.e., the amount of suspended particles presented in the liquid in the feed pipe for rotten tanks at biogas plants.

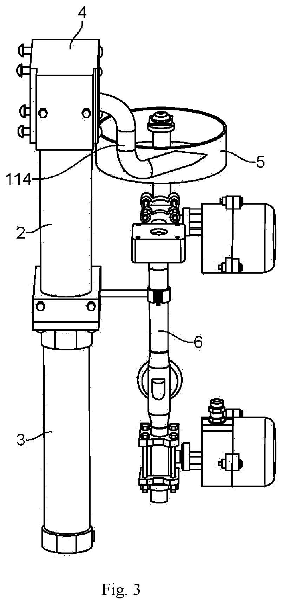

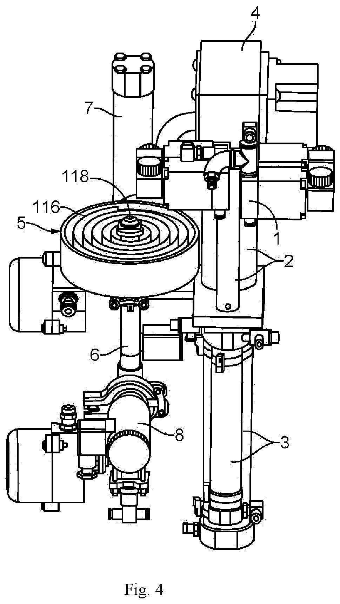

Referring to FIGs. **3-4****,** the components of the robot **100** are disclosed. The components of the robot **100** are securely and operatively disposed within the housing **104** (shown in FIG. **1**). The components include, but not limited to, one or more valve **1,** cylinders (**2** and **3**), a homogenizer **4,** a holding cell **5,** a pressure chamber **6,** a pneumatic cylinder **7,** and a sensor **8.** The valves **1** are securely disposed in the housing **104** for distributing a sample liquid/product and water into cylinders (**2** and **3**). The homogenizer **4** is securely and fluidly connected to the cylinders **3** and is configured to receive and homogenize the sample liquid and at the same time, the liquid is diluted in a mixing ratio determined by the stroke length of the cylinders **3.**

The holding cell **5 is** securely connected to the homogenizer **4** via a hose **114.** The holding cell **5** is configured to exhaust the liquid for removing large bubbles. In one embodiment, the holding cell **5** comprises a spiral **116,** which is configured to skim the liquid before running down to the sensor **8.** In one embodiment, the holding cell **5** further comprises a needle valve **118,** which is configured to regulate holding time for exhausting the liquid. The pressure chamber **6** is connected to the holding cell **5** and is configured to increase the pressure of the liquid above the equilibrium level, thereby condensing air bubbles in the liquid. The pneumatic cylinder **7** is operatively connected to the pressure chamber **6.** The pneumatic cylinder **7** further comprises a piston, which is configured to move to and fro within the pressure chamber **6,** thereby measuring the unresolved gases relative to the liquid based on the volume displaced by the piston in the pressure chamber **6.** In an exemplary embodiment, a magnetic inductive sensor is disposed on the cylinder which drives the piston. The piston runs past the sensor if there are many bubbles in the liquid. The current measurement cycle is interrupted and a new cycle will be initiated.

In one embodiment, the sensor **8** is securely and fluidly connected to a bottom portion of the pressure chamber **6,** configured to detect the volume displaced by the piston in the pressure chamber 6 and log or record the unresolved gases relative to the liquid. In one embodiment, the logged unresolved gases relative to the liquid could be displayed on the screen **108** (shown in FIG. **1**), which is mounted on the housing **104.** In one embodiment, the sensor **8** could be, but not limited to, an optical sensor and a magnetic inductive sensor. Analysis data is collected electronically and forms the basis for monitoring and adjustments in the process and adjustments may be made fully automatic without human interventions. In one embodiment, the robot **100** could measure fluorescence in the liquid. When measuring the fluorescence, the apparatus can regulate the liquid to a predetermined temperature. For example, the particles present in the liquid with fluorescent properties will change wavelength with temperature changes. Liquid refractive index/density also varies with temperature.

In one embodiment, the surface tension of the liquid squeezes the air bubbles with a radius of a few hundred nanometers so hard that the pressure in the air bubble could withstand a pressure difference of about 15 bar. Therefore, the robot **100** raises the pressure to a difference of more than 35 bar for 10 seconds before logging the measurement value. The robot **100** comminutes and homogenizes the particles in the liquid to optimize the repeatability of the measurement result. Liquid with uniform particles gives the highest data quality. The robot **100** is designed for fully automatic, liquid sample preparation, primarily for optical analysis and ensures optimal repeatability and human error is excluded. After analysis, the liquid can be returned to the process. All functions are controlled by a computer.

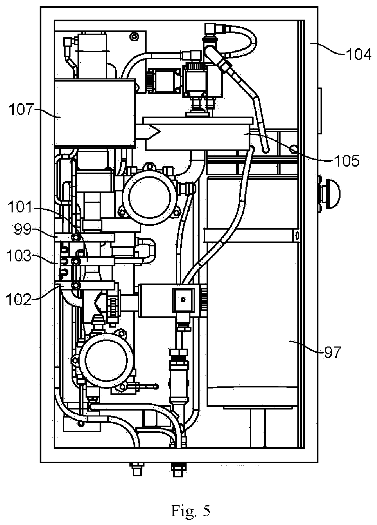

Referring to FIGs. **5-6****,** a water heater **97** securely disposed in the housing **104** of the robot **100** is disclosed. The robot **100** further comprises a water heater **97,** which is securely disposed in the housing **104.** The water heater **97** is configured to heat the water and distribute within the robot **100** via one or more hoses for cleaning i.e. with known turbidity. Then the measurement is logged on the flushing water, and if the turbidity is increasing it means that the turbidity meter's optics are contaminated and that hot flushing water must be used. In one example, the robot **100** is configured to measure the turbidity value i.e., the amount of suspended particles presented in the liquid in the feed pipe for rotten tanks at biogas plants. Turbidity is proportional to the number of particles presented in the liquid. The sampled liquid is diluted in a 1:1 ration to obtain half the measurement result. The robot multiplies the result by 2 before it is presented on the display. In one embodiment, the water heater **97** is powered via a power supply with 230 V. In one embodiment, a control unit **107** is disposed in the top portion of the housing **104** for controlling a drive unit or motor. In one embodiment, the robot **100** further comprises a manifold **103** with compressed air from a bulkhead passage **98.** In one embodiment, one or more hoses run from the manifold **103** to one or more pilot valves (**99, 101,** and **102**) for speed control. In one embodiment, a holding bracket **109** is provided for the manifold **103.** In one embodiment, the manifold **103** could be an air manifold.

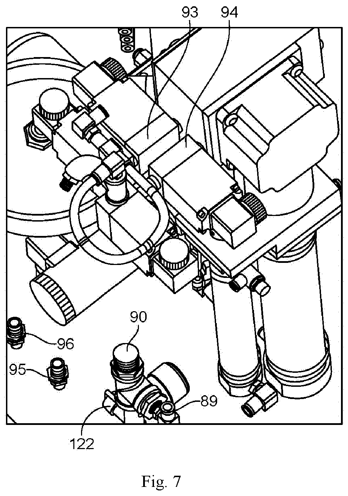

Referring to FIGs. **7-8****,** the valves (**91** and **92**) and hose connections of the robot **100** is disclosed. In one embodiment, one or more hoses are used for distributing cold and hot water, and compressed air within the robot **100.** In one embodiment, the hoses are securely connected to the plurality of valves (**91** and **92**) and the hose connections. In one embodiment, the valves (**91** and **92**) could be, but not limited to, 3-way valves. In one embodiment, transparent hoses are used to distribute liquids within the robot **100.** In one embodiment, a hose with an inlet runs from the water heater **97** and is securely fitted onto the member/nipple **90** over the safety valve **122,** and feeds cold water. In one embodiment, another hose runs from the member/nipple **89** over the safety valve **122** to a hose connection **83** at the bottom of the valve **92.** In one embodiment, another hose runs from the water heater **97** and is fixed or screwed onto the hose connection **75** with a 1/8" wrap.

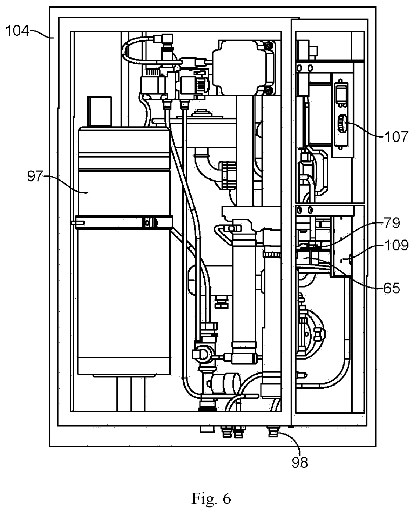

In one embodiment, another hose **123** runs from a hose connection **87** to a tee member **88** for distributing hot water via the valve **91.** In one embodiment, another hose runs from the tee member **88** down to a hose connection **79** (shown in FIG. **6**) of a ball valve **65** (shown in FIG. **6**). In one embodiment, another hose runs from a bulkhead entry **95** to a hose connection **82** at the bottom of the valve **91.** In one embodiment, another hose is a sewer pipe, which runs from the ball valve **65** to a bulkhead insertion item **96.**

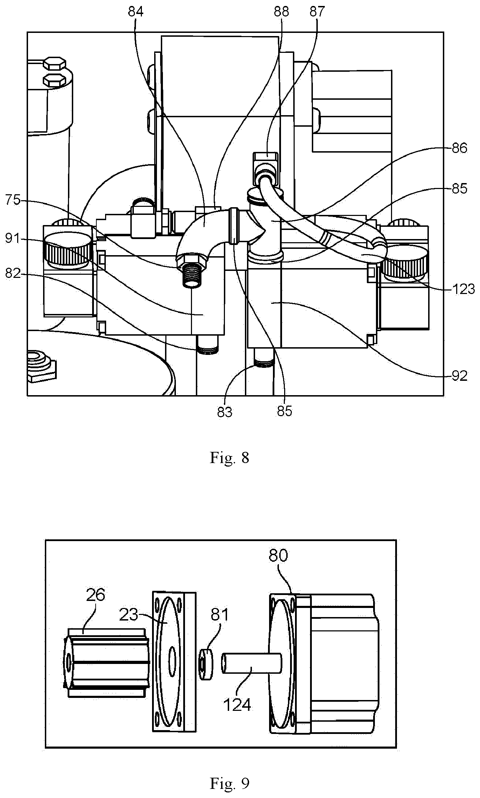

Referring to FIG. **9****,** a rotor **26** rotatably secured to a shaft **124** of a motor **80** is disclosed. In one embodiment, the motor **80** is securely disposed within the housing **104** of the robot **100.** In one embodiment, the rotor **26** is securely and rotatably connected to the shaft **124** of the motor **80** via a bearing **81** using a mounting plate **23.** In one embodiment, the motor **80** is configured to whip the liquids to ensure good and uniform mixing of a viscous product and water for dilution.

The advantages of the present invention include the robot **100** effectively measuring the amount of residual unresolved gas relative to the liquid by eliminating the air bubbles in the liquid and displaying results in real-time via the screen **108.** The robot **100** comminutes and homogenizes the particles in the liquid to optimize the repeatability of the measurement result. Liquid with uniform particles gives the highest data quality.

The foregoing examples have been provided merely for the purpose of explanation and are in no way to be construed as limiting of the present concept disclosed herein. While the concept has been described with reference to various embodiments, it is understood that the words, which have been used herein, are words of description and illustration, rather than words of limitation. Further, although the concept has been described herein with reference to particular means, materials, and embodiments, the concept is not intended to be limited to the particulars disclosed herein; rather, the concept extends to all functionally equivalent structures, methods and uses, such as are within the scope of the appended claims.

## Claims

1. An assay robot (**100**) for measuring unresolved gases in a liquid, wherein said measuring comprises condensing air bubbles in the liquid, and wherein said condensing air bubbles and measuring an amount of the unresolved gases in the liquid comprises providing the assay robot, comprising:
a housing (104);
cylinders (2, 3);
one or more valves (**1**) securely disposed in the housing (**104**), fluidly connected to and configured for distributing a sample of the liquid and water into the cylinders (**2**, **3**),
a sensor (**8**) configured to record the unresolved gases relative to the liquid, and
a pressure chamber (**6**) configured to increase the pressure of the sample liquid above the equilibrium level, thereby condensing air bubbles in the sample liquid,
a water heater (97), securely disposed within the housing (**2**), wherein the water heater (97) is connected to one or more hoses configured to distribute water within the assay robot (100);
a homogenizer (**4**) securely and fluidly connected to the cylinders (**3**), configured to receive and homogenize the sample liquid and water and at the same time, wherein the sample liquid is diluted in a mixing ratio determined by a stroke length of the cylinders (**3**);
a holding cell (**5**) securely connected to the homogenizer (**4**) via a hose (**114**), wherein the holding cell (**5**) is configured to exhaust the diluted sample liquid for removing large bubbles;
wherein the pressure chamber (**6**) is connected to the holding cell (**5**),
a pneumatic cylinder (**7**) operatively connected to the pressure chamber (**6**), wherein the pneumatic cylinder (**7**) comprises a piston, which is configured to move to and fro within the pressure chamber (**6**), thereby condensing air bubbles in the diluted sample liquid and permitting measurement of the unresolved gases relative to the liquid based on the volume displaced by the piston in the pressure chamber (**6**), and wherein
the sensor (**8**) is securely and fluidly connected to a bottom portion of the pressure chamber (**6**), and configured to detect the volume displaced by the piston in the pressure chamber (**6**).

2. The assay robot (**100**) of claim 1, further comprises a screen (**108**), securely mounted on the housing (**104**), wherein the screen (**108**) is configured to display the measurement value of the unresolved gases relative to the liquid.

3. The assay robot (**100**) of claim 1, wherein the holding cell (**5**) comprises a spiral (**116**), wherein the spiral (**116**) is configured to skim the diluted sample liquid before running down to the sensor (**8**).

4. The assay robot (**100**) of claim 1, wherein the holding cell (**5**) further comprises a needle valve (**118**), configured to regulate holding time for exhausting the diluted sample liquid.

5. The assay robot (**100**) of claim 1, wherein the sensor (**8**) is at least any one of an optical sensor and a magnetic inductive sensor.

6. The assay robot (**100**) of claim 1, wherein the housing (**104**) is securely and rotatably mounted on one or more supports (**110**) from a ground level, wherein the one or more supports (**110**) are securely anchored to the ground using an anchor (**112**).

7. The assay robot (**100**) of claim 1, further comprising a pair of handles (**106**), securely affixed to both sides of the housing (10**4**).

## Patentansprüche

1. Ein Analyse-Roboter (100) zum Messen nicht gelöster Gase in einer Flüssigkeit, wobei das Messen das Kondensieren von Luftblasen in der Flüssigkeit umfasst, und wobei das Kondensieren von Luftblasen und das Messen einer Menge der nicht gelösten Gase in der Flüssigkeit das Bereitstellen des Analyse-Roboters umfasst, wobei der Analyse-Roboter Folgendes umfasst:
ein Gehäuse (104);
Zylinder (2, 3);
ein oder mehrere Ventile (1), die fest im Gehäuse (104) angeordnet sind, fluidisch mit den Zylindern (2, 3) verbunden sind und dazu konfiguriert sind, eine Probe der Flüssigkeit und Wasser in die Zylinder (2, 3) zu verteilen,
einen Sensor (8), der dazu konfiguriert ist, die nicht gelösten Gase relativ zur Flüssigkeit zu erfassen, und
eine Druckkammer (6), die dazu konfiguriert ist, den Druck der Probenflüssigkeit über den Gleichgewichtspegel hinaus zu erhöhen, wodurch Luftblasen in der Probenflüssigkeit kondensiert werden,
einen Wassererhitzer (97), der fest innerhalb des Gehäuses (2) angeordnet ist, wobei der Wassererhitzer (97) mit einem oder mehreren Schläuchen verbunden ist, die dazu konfiguriert sind, Wasser innerhalb des Analyse-Roboters (100) zu verteilen;
einen Homogenisator (4), der fest und fluidisch mit den Zylindern (3) verbunden ist, dazu konfiguriert ist, die Probenflüssigkeit und Wasser gleichzeitig aufzunehmen und zu homogenisieren, wobei die Probenflüssigkeit in einem Mischungsverhältnis verdünnt wird, das durch einen Hubweg der Zylinder (3) bestimmt wird;
eine Haltezelle (5), die über einen Schlauch (114) fest mit dem Homogenisator (4) verbunden ist, wobei die Haltezelle (5) dazu konfiguriert ist, die verdünnte Probenflüssigkeit zur Entfernung großer Blasen abzuführen; wobei die Druckkammer (6) mit der Haltezelle (5) verbunden ist,
einen Pneumatikzylinder (7), der funktionell mit der Druckkammer (6) verbunden ist, wobei der Pneumatikzylinder (7) einen Kolben umfasst, der dazu konfiguriert ist, sich innerhalb der Druckkammer (6) hin- und herzubewegen, wodurch Luftblasen in der verdünnten Probenflüssigkeit kondensiert werden und eine Messung der nicht gelösten Gase relativ zur Flüssigkeit auf Basis des vom Kolben in der Druckkammer (6) verdrängten Volumens ermöglicht wird, und wobei
der Sensor (8) fest und fluidisch mit einem unteren Abschnitt der Druckkammer (6) verbunden ist und dazu konfiguriert ist, das vom Kolben in der Druckkammer (6) verdrängte Volumen zu erfassen.

2. Der Analyse-Roboter (100) nach Anspruch 1, der ferner einen Bildschirm (108) umfasst, der fest am Gehäuse (104) montiert ist, wobei der Bildschirm (108) dazu konfiguriert ist, den Messwert der nicht gelösten Gase relativ zur Flüssigkeit anzuzeigen.

3. Der Analyse-Roboter (100) nach Anspruch 1, wobei die Haltezelle (5) eine Spirale (116) umfasst, wobei die Spirale (116) dazu konfiguriert ist, die verdünnte Probenflüssigkeit vor dem Herunterlaufen zum Sensor (8) abzustreifen.

4. Der Analyse-Roboter (100) nach Anspruch 1, wobei die Haltezelle (5) ferner ein Nadelventil (118) umfasst, das dazu konfiguriert ist, die Haltezeit für das Abführen der verdünnten Probenflüssigkeit zu regulieren.

5. Der Analyse-Roboter (100) nach Anspruch 1, wobei der Sensor (8) mindestens einer von einem optischen Sensor und einem magnetisch-induktiven Sensor ist.

6. Der Analyse-Roboter (100) nach Anspruch 1, wobei das Gehäuse (104) fest und drehbar an einem oder mehreren Trägern (110) vom Boden aus montiert ist, wobei die ein oder mehreren Träger (110) mittels eines Ankers (112) fest im Boden verankert sind.

7. Der Analyse-Roboter (100) nach Anspruch 1, ferner umfassend ein Paar Handgriffe (106), die fest an beiden Seiten des Gehäuses (104) angebracht sind.

## Revendications

1. Un robot d'analyse (100) pour mesurer des gaz non dissous dans un liquide, ladite mesure comprenant la condensation de bulles d'air dans le liquide, et ladite condensation des bulles d'air et la mesure d'une quantité des gaz non dissous dans le liquide comprenant la mise en œuvre du robot d'analyse, comprenant :
un boîtier (104) ;
des cylindres (2, 3) ;
une ou plusieurs vannes (1) disposées de manière fixe dans le boîtier (104), connectées fluidiquement aux cylindres (2, 3) et configurées pour distribuer un échantillon de liquide et de l'eau dans lesdits cylindres (2, 3),
un capteur (8) configuré pour enregistrer les gaz non dissous relativement au liquide, et
une chambre de pression (6) configurée pour augmenter la pression du liquide échantillon au-dessus du niveau d'équilibre, de manière à condenser les bulles d'air dans le liquide échantillon,
un chauffe-eau (97), disposé de manière fixe à l'intérieur du boîtier (2), ledit chauffe-eau (97) étant relié à un ou plusieurs tuyaux configurés pour distribuer l'eau dans le robot d'analyse (100) ;
un homogénéiseur (4), fixé et connecté fluidiquement aux cylindres (3), configuré pour recevoir et homogénéiser simultanément le liquide échantillon et l'eau, le liquide échantillon étant dilué selon un rapport de mélange déterminé par la course des cylindres (3) ;
une cellule de maintien (5), fixée de manière rigide à l'homogénéiseur (4) par un tuyau (114), ladite cellule de maintien (5) étant configurée pour évacuer le liquide échantillon dilué afin d'éliminer les grandes bulles ; la chambre de pression (6) étant connectée à la cellule de maintien (5),
un vérin pneumatique (7) connecté fonctionnellement à la chambre de pression (6), ledit vérin pneumatique (7) comprenant un piston, lequel est configuré pour se déplacer d'avant en arrière à l'intérieur de la chambre de pression (6), de manière à condenser les bulles d'air dans le liquide échantillon dilué et à permettre la mesure des gaz non dissous relativement au liquide sur la base du volume déplacé par le piston dans la chambre de pression (6), et
le capteur (8) étant fixé et connecté fluidiquement à une partie inférieure de la chambre de pression (6), et configuré pour détecter le volume déplacé par le piston dans ladite chambre de pression (6).

2. Le robot d'analyse (100) selon la revendication 1, comprenant en outre un écran (108), monté de manière fixe sur le boîtier (104), l'écran (108) étant configuré pour afficher la valeur mesurée des gaz non dissous relativement au liquide.

3. Le robot d'analyse (100) selon la revendication 1, dans lequel la cellule de maintien (5) comprend une spirale (116), la spirale (116) étant configurée pour épurer le liquide échantillon dilué avant son écoulement vers le capteur (8).

4. Le robot d'analyse (100) selon la revendication 1, dans lequel la cellule de maintien (5) comprend en outre un robinet à pointeau (118), configuré pour réguler le temps de maintien de l'évacuation du liquide échantillon dilué.

5. Le robot d'analyse (100) selon la revendication 1, dans lequel le capteur (8) est au moins l'un d'un capteur optique et d'un capteur inductif magnétique.

6. Le robot d'analyse (100) selon la revendication 1, dans lequel le boîtier (104) est monté de manière fixe et pivotante sur un ou plusieurs supports (110) depuis le niveau du sol, lesdits un ou plusieurs supports (110) étant ancrés de manière fixe au sol à l'aide d'un ancrage (112).

7. Le robot d'analyse (100) selon la revendication 1, comprenant en outre une paire de poignées (106), fixées de manière rigide de chaque côté du boîtier (104).
